(19) Europäisches Patentamt
European Patent Office
Office européen des brevets

(11) **EP 4 032 429 A1**

(12) **EUROPEAN PATENT APPLICATION**
published in accordance with Art. 153(4) EPC

(43) Date of publication:
**27.07.2022 Bulletin 2022/30**

(21) Application number: **20864621.6**

(22) Date of filing: **16.09.2020**

(51) International Patent Classification (IPC):
**A45D 34/02** $^{(2006.01)}$    **F16L 11/06** $^{(2006.01)}$

(52) Cooperative Patent Classification (CPC):
**C08F 210/02; C08F 214/26; C08F 214/28;**
A45D 34/02; F16L 11/06

(86) International application number:
**PCT/JP2020/035090**

(87) International publication number:
**WO 2021/054364 (25.03.2021 Gazette 2021/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 20.09.2019 JP 2019171294
16.10.2019 JP 2019189130
22.01.2020 JP 2020008109

(71) Applicant: **Daikin Industries, Ltd.**
**Osaka-shi, Osaka 530-8323 (JP)**

(72) Inventors:
• **KUWAJIMA, Yuuki**
  **Osaka-shi, Osaka 530-8323 (JP)**
• **FUKAGAWA, Ryouichi**
  **Osaka-shi, Osaka 530-8323 (JP)**
• **ISAKA, Tadaharu**
  **Osaka-shi, Osaka 530-8323 (JP)**
• **SHIMADA, Hiroyuki**
  **Osaka-shi, Osaka 530-8323 (JP)**

(74) Representative: **Hoffmann Eitle**
**Patent- und Rechtsanwälte PartmbB**
**Arabellastraße 30**
**81925 München (DE)**

(54) **TUBE AND FRAGRANCE PRODUCT**

(57)    There is provided a tube for transferring a liquid having a refractive index of 1.35 to 1.41 wherein the tube contains a fluoropolymer, and the fluoropolymer has a melt flow rate of 3 to 150 g/10 min, and a light transmittance at a wavelength of 300 nm of 85% or higher.

EP 4 032 429 A1

**Description**

TECHNICAL FIELD

The present disclosure relates to a tube to be used for transferring a liquid having a refractive index of 1.35 to 1.41, and a fragrance product using such a tube.

BACKGROUND ART

Liquid fragrances are sold in the form of being accommodated in transparent containers equipped with a spray pump or a dispenser pump, as fragrance products to general consumers. For example, Patent Document 1 discloses a tube composed of a fluoropolymer as a tube for feeding a liquid fragrance to a spray pump or a dispenser pump.

RELATED ART

PATENT DOCUMENT

Patent Document 1: Japanese Patent Laid-Open No. 2014-12185

SUMMARY OF THE INVENTION

PROBLEM TO BE SOLVED BY THE INVENTION

The present disclosure has an object to provide a tube low in visibility when the tube is immersed in a liquid having a refractive index of 1.35 to 1.41.

MEANS FOR SOLVING THE PROBLEM

According to the present disclosure, provided is a tube for transferring a liquid having a refractive index of 1.35 to 1.41, the tube comprising a fluoropolymer wherein the fluoropolymer has a melt flow rate of 3 to 150 g/10 min and a light transmittance at a wavelength of 300 nm of 85% or higher.
It is preferable that in the tube in the present disclosure, the fluoropolymer has a refractive index of 1.37 to 1.39.
It is preferable that in the tube in the present disclosure, the fluoropolymer has a haze value of 0.01 to 5.0%.
It is preferable that in the tube in the present disclosure, the fluoropolymer has a number of fish eyes of 5,000/$m^2$ or less.
It is preferable that in the tube in the present disclosure, the fluoropolymer has a yellow index value of 5 or lower.
It is preferable that in the tube in the present disclosure, the fluoropolymer has a tensile elastic modulus of 400 MPa or higher.
It is preferable that the tube in the present disclosure has an outer diameter of 0.5 to 5.0 mm.
It is preferable that in the tube in the present disclosure, the fluoropolymer is at least one selected from the group consisting of an ethylene/tetrafluoroethylene/hexafluoropropylene copolymer, a polychlorotrifluoroethylene, a chlorotrifluoroethylene-based copolymer, a vinylidene fluoride/tetrafluoroethylene copolymer, a tetrafluoroethylene/hexafluoropropylene copolymer, and a tetrafluoroethylene/hexafluoropropylene/vinylidene fluoride copolymer; and it is more preferable that the fluoropolymer is an ethylene/tetrafluoroethylene/hexafluoropropylene copolymer.
It is preferable that in the tube in the present disclosure, the fluoropolymer has a content of each of Na, Cu, K, Ca, Fe and Zn of 1.0 µg/1 g or lower as measured by an ashing method.
It is preferable that in the tube in the present disclosure, the liquid is a liquid fragrance.
According to the present disclosure, a container comprising the tube is provided.
According to the present disclosure, provided is a fragrance product comprising a transparent container accommodating the liquid having a refractive index of 1.35 to 1.41 and a tube for sucking up the liquid.

EFFECTS OF INVENTION

According to the present disclosure, there can be provided a tube low in visibility when the tube is immersed in a liquid having a refractive index of 1.35 to 1.41.

DESCRIPTION OF EMBODIMENTS

Hereinafter, specific embodiments in the present disclosure will be described in detail, but the present disclosure is not any more limited to the following embodiments.

A tube in the present disclosure is a tube for transferring a liquid having a refractive index of 1.35 to 1.41, the tube comprising a fluoropolymer wherein the fluoropolymer has a melt flow rate of 3 to 150 g/10 min and a light transmittance at a wavelength of 300 nm of 85% or higher.

The tube in the present disclosure is a tube for transferring a liquid having a refractive index of 1.35 to 1.41; and the liquid to which the tube is applied may have a refractive index in the range of 1.35 to 1.41, and examples of such a liquid include liquid fragrances. The liquid fragrances are liquids containing components diffusing aroma, and usually contain perfume components. The liquid fragrances are constituted, for example, by suitably blending perfume components constituting base notes, perfume components constituting middle notes and perfume components constituting top notes. The liquid fragrances are classified, depending on content proportions of the perfume components, for example, into perfume extracts, perfumes, eau de toilette, eau de cologne, aftershaves. Here, the refractive index can be measured at 25°C by using an Abbe's refractometer with the sodium D-line as the light source.

Liquid fragrances as one example of liquids having a refractive index of 1.35 to 1.41 are often sold in the form of being accommodated in transparent containers equipped with a spray pump or a dispenser pump, as fragrance products to general consumers. Then, the spray pump or dispenser pump is equipped with a tube (a tube for transferring the liquid fragrance) for feeding the liquid fragrance to the spray pump or dispenser pump; and such a tube is accommodated together with the liquid fragrance in the container in the state of being immersed in the liquid fragrance. With regard to fragrance products containing the liquid fragrance, being excellent in the aesthetic property of their appearance is regarded as being suitable; hence, it is desired that the tube to be used therein is, in the state of being immersed in the liquid fragrance, low in visibility, that is, in a state of being hardly visible, particularly in a state of being substantially invisible (in a state of being viewed as being tubeless at first sight and being invisible unless cautiously viewed).

By contrast, the present inventors have found that a tube containing a fluoropolymer having a melt flow rate of 3 to 150 g/10 min and a light transmittance at a wavelength of 300 nm of 85% or higher can be low in visibility. More specifically, it has been found that the tube can be low in visibility when the tube is immersed in a liquid, such as a liquid fragrance, having a refractive index of 1.35 to 1.41. The tube in the present disclosure may be a tube for transferring a liquid having a refractive index of 1.35 to 1.41; hence, it is a matter of course that the tube can also be used for transferring a liquid, other than a liquid fragrance, having a refractive index of 1.35 to 1.41.

The tube in the present disclosure comprises a fluoropolymer having a melt flow rate of 3 to 150 g/10 min, and a light transmittance at a wavelength of 300 nm of 85% or higher.

The fluoropolymer used in the present disclosure has a melt flow rate (MFR) of 3 to 150 g/10 min. The melt flow rate of the fluoropolymer is preferably 8 g/10 min or higher, more preferably 12 g/10 min or higher, still more preferably 20 g/10 min or higher and especially preferably 25 g/10 min or higher, and preferably 150 g/10 min or lower, more preferably 80 g/10 min or lower, still more preferably 70 g/10 min or lower, further still more preferably 60 g/10 min or lower and especially preferably 50 g/10 min or lower. When the melt flow rate is in this range, the generation of the melt fracture in molding into the tube can effectively be suppressed and there can thereby effectively be suppressed the increase in the visibility and the degradation of the low visibility due to the light refraction caused by irregularities by the generation of the melt fracture, resulting in that an obtained tube can be low in visibility. In particular, according to the present disclosure, with the melt flow rate in the above range, even in the case where molding into the tube is carried out in a relatively high speed and also even in the case of molding into the tube small in diameter, the generation of the melt fracture can effectively be suppressed, thereby enabling contribution also to the improvement in the productivity. On the other hand, with the melt flow rate of the fluoropolymer being too low, in molding into the tube, the melt fracture ends in generating on the outer surface or the inner surface of the tube, thereby resulting in making the visibility high and making the tube inferior in low visibility. On the other hand, with the melt flow rate of the fluoropolymer being too high, molding into the tube ends in becoming difficult.

The melt flow rate of the fluoropolymer can be measured according to ASTM D1238 by using a melt indexer. Set values of the measurement temperature, the load and the like may be determined by reference to the standards (for example, ASTM D2116) of individual fluoropolymers.

The fluoropolymer used in the present disclosure has a light transmittance at a wavelength of 300 nm of 85% or higher. The light transmittance at a wavelength of 300 nm is preferably 88% or higher, more preferably 90% or higher and still more preferably 91% or higher, and preferably 98% or lower, more preferably 96% or lower and still more preferably 95% or lower. With the light transmittance at a wavelength of 300 nm in this range, an obtained tube is high in transparency and low in visibility. With the light transmittance at a wavelength of 300 nm being too low, an obtained tube ends in becoming one low in transparency and high in visibility, and making the tube inferior in low visibility. The light transmittance at a wavelength of 300 nm of the fluoropolymer can be measured for a sheet of 0.1 mm in thickness of a fluoropolymer fabricated by the following method, by using a spectrophotometer at a wavelength of 300 nm.

(Method for fabricating a sheet of a fluoropolymer)

Resin pellets (pellets of a fluoropolymer) are charged in a metal mold of 120 mm in diameter, set on a press machine heated at 300°C, and melt pressed at a pressure of about 2.9 MPa to thereby obtain a sheet of 0.1 mm in thickness of the fluoropolymer.

The refractive index of the fluoropolymer used in the present disclosure is not limited, and is preferably 1.37 to 1.39 and more preferably 1.38 to 1.39. With the refractive index of the fluoropolymer in this range, the difference in refractive index between the tube in the present disclosure and a liquid to which the tube in the present disclosure is applied and having a refractive index of 1.35 to 1.41 can be small, thereby enabling an obtained tube to be one lower in visibility. The refractive index of the fluoropolymer can be measured at 25°C by using an Abbe's refractometer with the sodium D-line as the light source, and can be measured by using the sheet of a fluoropolymer fabricated in the above method.

The haze value of the fluoropolymer used in the present disclosure is not limited, and is preferably 0.01 to 5.0%, more preferably 0.05 to 3.0% and still more preferably 0.1 to 1.0%. With the haze value of the fluoropolymer in this range, an obtained tube can be lower in visibility. The haze value of the fluoropolymer can be measured for the sheet of 0.1 mm in thickness of a fluoropolymer fabricated by the above method by using a haze meter according to ASTM D1003.

The number of fish eyes of the fluoropolymer used in the present disclosure is not limited, and is preferably $5,000/m^2$ or less, more preferably $3,000/m^2$ or less, still more preferably $1,000/m^2$ or less and most preferably $500/m^2$ or less. With the number of fish eyes of the fluoropolymer in this range, an obtained tube can be lower in visibility. The above fish eyes are foreign matter present as impurities in fluoropolymers because of having largely different molecular weights and compositions from those of the target fluoropolymers, and in molding into films, can be visually recognized as white opaque portions or protrusions. In particular, in fluoropolymers, components having unusually high molecular weights, components produced by recombination or crosslinking by heat in molding, or the like make the cause of the fish eyes. Therefore, by preventing the production of these components, the fish eyes can be reduced. The number of the fish eyes of the fluoropolymer can be measured by the following method. That is, for a molded film of 0.05 to 0.06 mm in thickness, fish eyes are detected by using a surface inspection apparatus (manufactured by Mitsubishi Rayon Co., Ltd., LSC-3100V); for detected fish eyes, the number of fish eyes having a size of 50 $\mu$m or larger in one side is measured and the number of the fish eyes per 1 $m^2$ is determined; and the number is taken as the number of fish eyes (unit: the number/$m^2$) of the fluoropolymer.

The yellow index of the fluoropolymer used in the present disclosure is not limited, and is preferably 5 or lower, more preferably 2 or lower, still more preferably 0 or lower and most preferably -3 or lower. With the yellow index of the fluoropolymer in this range, an obtained tube can be lower in visibility. A method of making the yellow index of the fluoropolymer in the above range is not limited, and examples thereof include a method of adjusting the kinds of polymerization units and the composition of polymerization units constituting the fluoropolymer, and a method of adjusting the kinds and the amounts of polymerization raw materials to be used in production of the fluoropolymer. The yellow index of the fluoropolymer can be measured by using a colorimeter (manufactured by Nippon Denshoku Industries Co., Ltd., ZE-6000) with its dedicated cell filled with resin pellets (pellets of the fluoropolymer) according to JIS K7373.

The tensile elastic modulus of the fluoropolymer used in the present disclosure is not limited, and is preferably 150 MPa or higher, more preferably 400 MPa or higher, still more preferably 500 MPa or higher, especially preferably 700 MPa or higher and most preferably 850 MPa or higher. With the tensile elastic modulus of the fluoropolymer in this range, an obtained tube can have better insertability. The tensile elastic modulus of the fluoropolymer can be measured by the following method. That is, resin pellets (pellets of the fluoropolymer) are charged in a metal mold, set on a press machine heated at 240 to 300°C, and melt pressed at a pressure of 3 MPa to thereby obtain a sheet of 2 mm in thickness of the fluoropolymer. Then, by using the obtained sheet of the fluoropolymer, the tensile elastic modulus is measured according to ASTM D638 under the condition of 25°C and 50 mm/min, and the result obtained is taken as a value of the tensile elastic modulus.

The crystallinity of the fluoropolymer used in the present disclosure is not limited, and is preferably 13% to 60%, more preferably 14% to 50%, still more preferably 15% to 40% and most preferably 15 to 35%. With the crystallinity of the fluoropolymer in this range, an obtained tube can be lower in visibility. A method of making the crystallinity of the fluoropolymer in the above range is not limited, and examples thereof include a method of adjusting the kinds of polymerization units and the composition of polymerization units constituting the fluoropolymer. The crystallinity of the fluoropolymer can be determined by carrying out wide-angle X-ray diffractometry in the range of 5 to 40 degrees in scanning angle by using an X-ray diffractometer (manufactured by Rigaku Corp., SmartLab), and using the measurement result and the following expression.

Crystallinity of the fluoropolymer (%) = 100 × (a peak area derived from a crystal of the fluoropolymer)/(a whole peak area)

The content of each of Na, Cu, K, Ca, Fe and Zn as measured by an ashing method of the fluoropolymer used in the present disclosure is preferably 1.0 μg/1 g or lower, more preferably 0.8 μg/1 g or lower, still more preferably 0.6 μg/1 g or lower and especially preferably 0.5 μg/1 g or lower. With the content of each of Na, Cu, K, Ca, Fe and Zn as measured by the ashing method in the above range, the influence on coloring to be imparted to the tube in the present disclosure can be suppressed, and there can effectively be suppressed the dissolving-out of these metals from the tube in the present disclosure into a liquid having a refractive index of 1.35 to 1.41, thus enabling the adverse influence of these metals to be suitably eliminated.

As a method using an ashing method of measuring the content of each of Na, Cu, K, Ca, Fe and Zn, there can be used a method of ashing the fluoropolymer in a cuvette in an atomizing part of an atomic absorption spectrophotometer and measuring the content of each of Na, Cu, K, Ca, Fe and Zn by using the atomic absorption spectrophotometer, a method of weighing the fluoropolymer in a platinum crucible, ashing the fluoropolymer by using a gas burner or an electric oven, dissolving the resultant ash content in an acid, and thereafter measuring the content of each of Na, Cu, K, Ca, Fe and Zn by an ICP atomic emission analyzer or a flameless atomic absorption spectrophotometer, or other methods.

The initial pyrolysis temperature, which is a temperature when 1% by mass of the mass of the fluoropolymer used in the present disclosure is lost, is preferably 395°C or higher, more preferably 400°C or higher, still more preferably 410°C or higher and further still more preferably 420°C or higher.

The fluoropolymer used in the present disclosure may have a melt flow rate and a light transmittance at a wavelength of 300 nm in the above ranges, and is not limited, and preferable is at least one selected from the group consisting of an ethylene/tetrafluoroethylene [TFE]/a hexafluoropropylene [HFP] copolymer, a polychlorotrifluoroethylene [PCTFE], a chlorotrifluoroethylene [CTFE]-based copolymer, a vinylidene fluoride [VdF]/tetrafluoroethylene [TFE] copolymer, a tetrafluoroethylene [TFE]/hexafluoropropylene [HFP] copolymer, and a tetrafluoroethylene [TFE]/hexafluoropropylene [HFP]/vinylidene fluoride [VdF] copolymer.

These fluoropolymers may be used by being mixed in optional proportions of two or more kinds thereof, and in this case, may be in a form in which two or more kinds of fluoropolymers having a different kind of monomer unit are mixed, may be in a form in which two or more kinds of fluoropolymers having the same monomer unit in a different content proportion are mixed, may be in a form in which two or more kinds of fluoropolymers having the same monomer unit in the same content proportion are mixed, or may be in a form in which these are combined and mixed; and preferable is the form in which two or more kinds of fluoropolymers having the same monomer unit in the same content proportion are mixed.

The content proportion of the fluoropolymer in the tube of the present invention is preferably 90% by weight or higher, more preferably 95% by weight or higher, still more preferably 98% by weight or higher, especially preferably 99% by weight or higher and most preferably 100% by weight. That is, it is most preferable that the tube of the present invention is constituted substantially only of the fluoropolymer. In this case, the fluoropolymer may be one containing trace amounts of impurities and the like contained inevitably.

As the fluoropolymer, particularly since an obtained tube can be sufficiently low in visibility, preferable are the ethylene/TFE/HFP copolymer, the CTFE-based copolymer, the TFE/HFP copolymer, and the TFE/HFP/VdF copolymer; and more preferable is the ethylene/TFE/HFP copolymer.

The ethylene/TFE/HFP copolymer is a copolymer containing an ethylene unit, a TFE unit and an HFP unit. It is preferable that the ethylene/TFE/HFP copolymer contains 30 to 70% by mol of the ethylene unit, 20 to 55% by mol of the TFE unit and 1 to 30% by mol of the HFP unit; it is more preferable that, 33 to 60% by mol of the ethylene unit, 25 to 52% by mol of the TFE unit and 4 to 25% by mol of the HFP unit; and it is still more preferable that, 35 to 55% by mol of the ethylene unit, 30 to 47% by mol of the TFE unit and 8 to 20% by mol of the HFP unit.

It is preferable that the ethylene/TFE/HFP copolymer further contains a polymerization unit of an ethylenically unsaturated monomer (excluding ethylene, TFE and HFP). The content of the polymerization unit of the ethylenically unsaturated monomer, with respect to the whole of the polymerization units, may be 0.1 to 10% by mol, may be 0.1 to 5% by mol, may be 0.2 to 1% by mol, or may be 0.3 to 0.8% by mol.

The ethylenically unsaturated monomer is not limited as long as being a monomer copolymerizable with ethylene, TFE and HFP, but preferable is at least one selected from the group consisting of ethylenically unsaturated monomers (excluding TFE and HFP) represented by the following formulas (1) and (2).

Formula (1): $CX^1X^2=CX^3 (CF_2)_nX^4$

wherein $X^1$, $X^2$, $X^3$ and $X^4$ are the same or different, and denote H, F or Cl; and n denotes an integer of 0 to 8.

Formula (2): $CF_2=CF-ORf^1$

wherein $Rf^1$ denotes an alkyl group having 1 to 3 carbon atoms or a fluoroalkyl group having 1 to 3 carbon atoms.

As an ethylenically unsaturated monomer represented by the formula (1), preferable is at least one selected from the group consisting of $CF_2=CFCl$, the following formula:

$$CH_2=CF-(CF_2)_nX^4 \cdots \qquad (3)$$

wherein $X^4$ and n are the same as in the above, and the following formula (4):

$$CH_2=CH-(CF_2)_nX^4 \cdots \qquad (4)$$

wherein $X^4$ and n are the same as in the above; more preferable is at least one selected from the group consisting of $CF_2=CFCl$, $CH_2=CFCF_3$, $CH_2=CH-C_4F_9$, $CH_2=CH-C_6F_{13}$ and $CH_2=CF-C_3F_6H$; still more preferable is at least one selected from the group consisting of $CF_2=CFCl$, $CH_2=CH-C_6F_{13}$, $CH_2=CFCF_3$ and $CH_2=CF-C_3F_6H$; and especially preferable is $CH_2=CF-C_3F_6H$ (that is, 2,3,3,4,4,5,5-heptafluoro-1-pentene ($CH_2=CFCF_2CF_2CF_2H$)).

As an ethylenically unsaturated monomer represented by the formula (2), preferable is at least one selected from the group consisting of $CF_2=CF-OCF_3$, $CF_2=CF-OCF_2CF_3$ and $CF_2=CF-OCF_2CF_2CF_3$.

The PCTFE is a homopolymer of chlorotrifluoroethylene [CTFE].

It is preferable that the CTFE-based copolymer contains a polymerization unit (CTFE unit) derived from CTFE, and a copolymerization unit derived from at least one monomer selected from the group consisting of TFE, HFP, perfluoro(alkyl vinyl ether)s [PAVE], VdF, vinyl fluoride, hexafluoroisobutene, monomers represented by $CH_2=CX^5(CF_2)_mX^6$ (wherein $X^5$ is H or F; $X^6$ is H, F or Cl; and m is an integer of 1 to 10), ethylene, propylene, 1-butene, 2-butene, vinyl chloride and vinylidene chloride. It is more preferable that the CTFE-based copolymer is a perhalopolymer.

It is more preferable that the CTFE-based copolymer contains the CTFE unit, and a polymerization unit derived from at least one selected from the group consisting of TFE, HFP and PAVE; and it is still more preferable that the CTFE-based copolymer is composed substantially only of these polymerization units. It is also preferable that there is contained substantially no monomer having a CH bond of ethylene, vinylidene fluoride, vinyl fluoride and the like.

It is preferable that the CTFE-based copolymer has the CTFE unit of 10 to 90% by mol of the whole of the polymerization units.

As the CTFE-based copolymer, especially preferable is one containing the CTFE unit, the TFE unit and a monomer ($\alpha$) unit derived from a monomer ($\alpha$) copolymerizable with these.

The "CTFE unit" and the "TFE unit" are, in the molecular structure of the CTFE-based copolymer, a moiety ($-CFCl-CF_2-$) derived from CTFE and a moiety ($-CF_2-CF_2-$) derived from TFE, respectively; and the "monomer ($\alpha$) unit" is, in the molecular structure of the CTFE-based copolymer, similarly a moiety made by addition of the monomer ($\alpha$).

The monomer ($\alpha$) is not limited as long as being a monomer copolymerizable with CTFE and TFE, and includes ethylene (Et), vinylidene fluoride (VdF), PAVE represented by $CF_2=CF-ORf^2$ (wherein $Rf^2$ is a perfluoroalkyl group having 1 to 8 carbon atoms), vinyl monomers represented by $CX^7X^8=CX^9(CF_2)_pX^{10}$ (wherein $X^7$, $X^8$ and $X^9$ are the same or different, and each of $X^7$, $X^8$ and $X^9$ is a hydrogen atom or a fluorine atom; $X^{10}$ is a hydrogen atom, a fluorine atom or a chlorine atom; and p is an integer of 1 to 10), and alkyl perfluorovinyl ether derivatives represented by $CF_2=CF-O-Rf^3$ (wherein $Rf^3$ is a perfluoroalkyl group having 1 to 5 carbon atoms).

As the alkyl perfluorovinyl ether derivative, preferable is one in which $Rf^3$ is a perfluoroalkyl group having 1 to 3 carbon atoms; and more preferable is $CF_2=CF-OCF_2-CF_2CF_3$.

As the monomer ($\alpha$), among these, preferable is at least one selected from the group consisting of PAVE, the above vinyl monomers and alkyl perfluorovinyl ether derivatives; more preferable is at least one selected from the group consisting of PAVE and HFP; and especially preferable is PAVE. That is, a CTFE/TFE/PAVE copolymer is especially preferable; and as the CTFE/TFE/PAVE copolymer, preferable is a copolymer composed substantially only of CTFE, TFE and PAVE.

With regard to the ratio of the CTFE unit and the TFE unit in the CTFE-based copolymer, preferably, with respect to 15 to 90% by mol of the CTFE unit, the TFE unit is 85 to 10% by mol; and more preferably, the CTFE unit is 15 to 50% by mol and the TFE unit is 85 to 50% by mol. Also preferable is a CTFE-based copolymer constituted of 15 to 25% by mol of the CTFE unit and 85 to 75% by mol of the TFE unit.

The CTFE-based copolymer is preferably one in which the total of the CTFE unit and the TFE unit is 90 to 99.9% by mol and the monomer ($\alpha$) unit is 0.1 to 10% by mol. When the monomer ($\alpha$) unit is less than 0.1% by mol, the CTFE-based copolymer is liable to be inferior in the moldability and the crack resistance; and when exceeding 10% by mol, the CTFE-based copolymer is likely to be inferior in the mechanical properties and the visibility.

In the CTFE/TFE/PAVE copolymer, the Example of the PAVE includes perfluoro(methyl vinyl ether) (PMVE), perfluoro(ethyl vinyl ether) (PEVE), perfluoro(propyl vinyl ether) (PPVE) and perfluoro(butyl vinyl ether); among these, preferable is at least one selected from the group consisting of PMVE, PEVE and PPVE.

In the CTFE/TFE/PAVE copolymer, the PAVE unit is preferably 0.5% by mol or more of the whole of the polymerization units, and preferably 5% by mol or less thereof.

The contents of the constituent units such as the CTFE unit are values obtained by carrying out $^{19}$F-NMR analysis.

The VdF/TFE copolymer is a copolymer containing a VdF unit and a TFE unit. With regard to the content proportions of the VdF unit and the TFE unit, since an obtained tube can be lower in visibility, the molar ratio of the VdF/TFE units is preferably 50/50 to 99/1, more preferably 60/40 to 98/2, still more preferably 70/30 to 97/3, especially preferably 74/26 to 96/4 and most preferably 78/22 to 96/4.

It is preferable that the VdF/TFE copolymer further contains a polymerization unit of an ethylenically unsaturated monomer (excluding VdF and TFE). The content of the polymerization unit of the ethylenically unsaturated monomer, with respect to the whole of the polymerization units, may be 0 to 50% by mol, may be 0 to 40% by mol, may be 0 to 30% by mol, may be 0 to 15% by mol, or may be 0 to 5% by mol.

The ethylenically unsaturated monomer is not limited as long as being a monomer copolymerizable with VdF and TFE, but preferable is at least one selected from the group consisting of ethylenically unsaturated monomers (excluding VdF and TFE) represented by the above formulas (1) and (2); and the compounds exemplified as preferable compounds in the above description can suitably be used.

The TFE/HFP copolymer is a copolymer containing a TFE unit and an HFP unit. With regard to the ratio of the TFE unit and the HFP unit, preferable is a TFE/HFP copolymer constituted of, with respect to 60 to 99% by mol of the TFE unit, 40 to 1% by mol of the HFP unit; more preferable is a TFE/HFP copolymer constituted of, with respect to 70 to 97% by mol of the TFE unit, 30 to 3% by mol of the HFP unit; and still more preferable is a TFE/HFP copolymer constituted of, with respect to 75 to 93% by mol of the TFE unit, 25 to 7% by mol of the HFP unit. It is desirable that the TFE/HFP copolymer further contains an ethylenically unsaturated monomer (excluding ethylene, TFE, HFP and VdF).

The ethylenically unsaturated monomer is not limited as long as being a monomer copolymerizable with TFE and HFP, but preferable is at least one selected from the group consisting of ethylenically unsaturated monomers (excluding TFE, HFP and VdF) represented by the following formulas (5) and (6).

$$\text{Formula (5):} \qquad CX^{11}X^{12}=CX^{13}(CF_2)_qX^{14}$$

wherein $X^{11}$, $X^{12}$, $X^{13}$ and $X^{14}$ are the same or different, and denote H, F, Cl or Br; and q denotes an integer of 0 to 8.

$$\text{Formula (6)} \qquad : CF_2=CF-ORf^4$$

wherein $Rf^4$ denotes an alkyl group having 1 to 3 carbon atoms or a fluoroalkyl group having 1 to 3 carbon atoms.

As an ethylenically unsaturated monomer represented by the formula (5), preferable is at least one selected from the group consisting of $CF_2=CFCl$, the following formula (7):

$$CH_2=CF-(CF_2)_qX^{14} \cdots \qquad (7)$$

wherein $X^{14}$ and q are the same as in the above, and the following formula (8):

$$CH_2=CH-(CF_2)_qX^{14} \cdots \qquad (8)$$

wherein $X^{14}$ and q are the same as in the above; more preferable is at least one selected from the group consisting of $CHF=CHF$, $CF_2=CFCl$, $CF_2=CFCl$, $CH_2=CF-CF_3$, $CH_2=CH-C_4F_9$, $CH_2=CH-C_6F_{13}$, $CH_2=CF-C_3F_6H$, $CF_2=CHBr$, $CH_2=CH-CF_2CF_2Br$, $CF_2=CFBr$, $CH_2=CH-CF_2Br$, and perfluoroalkyl vinyl ethers; still more preferable is at least one selected from the group consisting of $CF_2=CFCl$, $CH_2=CH-C_6F_{13}$, $CH_2=CF-CF_3$, $CH_2=CF-C_3F_6H$, $CF_2=CHBr$, $CH_2=CH-CF_2CF_2Br$, $CF_2=CFBr$, perfluoro(methyl vinyl ether), perfluoro(ethyl vinyl ether) and perfluoro(propyl vinyl ether); especially preferable is at least one selected from the group consisting of $CH_2=CF-C_3F_6H$ (that is, 2,3,3,4,4,5,5-heptafluoro-1-pentene ($CH_2=CFCF_2CF_2CF_2H$)), $CF_2=CHBr$, $CH_2=CH-CF_2CF_2Br$ and perfluoro (propyl vinyl ether); and most preferable is perfluoro(propyl vinyl ether). That is, as the TFE/HFP copolymer, a TFE/HFP/perfluoro(propyl vinyl ether) is preferable.

The TFE/HFP/VdF copolymer is a copolymer containing a TFE unit, an HFP unit and a VdF unit. Since the TFE/HFP/VdF copolymer, when the VdF content is high, is excellent in flexibility, with regard to the copolymerization proportions (ratios in % by mol) of TFE/HFP/VdF, it is preferable that 25 to 75% by mol of the TFE unit, 1 to 15% by mol of the HFP unit and 24 to 70% by mol of the VdF unit are contained; it is more preferable that 30 to 55% by mol of the TFE unit, 3 to 12% by mol of the HFP unit and 35 to 65% by mol of the VdF unit are contained; and it is most preferable that 30 to 40% by mol of the TFE unit, 3 to 10% by mol of the HFP unit and 55 to 65% by mol of the VdF unit are contained. The TFE/HFP/VdF copolymer may further contain 0 to 20% by mol of another monomer. As the another monomer, preferable is at least one selected from the group consisting of ethylenically unsaturated monomers (excluding TFE, HFP and VdF) represented by the above formulas (5) and (6); more preferable is at least one selected from the group consisting of fluorine-containing monomers such as perfluoro(methyl vinyl ether), perfluoro(ethyl vinyl ether), perfluoro(propyl vinyl ether), chlorotrifluoroethylene, 2-chloropentafluoropropene and perfluorinated vinyl ethers (for example, perfluoroalkoxy vinyl ethers such as $CF_3OCF_2CF_2CF_2OCF=CF_2$), perfluoroalkyl vinyl ethers, perfluoro-1,3-butadiene, trifluoroethylene, hexafluoroisobutene, vinyl fluoride, ethylene, propylene and alkyl vinyl ethers; and most preferable are perfluoro(methyl vinyl ether), perfluoro(ethyl vinyl ether) and perfluoro(propyl vinyl ether).

Since the TFE/HFP/VdF copolymer, when the VdF unit content is low, is excellent in chemical resistance, with regard to the copolymerization proportions (ratios in % by mol) of the TFE unit, the HFP unit and the VdF unit, it is preferable that TFE/HFP/VdF is 55 to 95/0.1 to 10/0.1 to 35; being 55 to 90/1 to 10/1 to 35 (in molar ratio) is more preferable; being 55 to 85/3 to 10/2 to 35 (in molar ratio) is still more preferable; and being 60 to 85/5 to 10/3 to 35 (in molar ratio) is most preferable. The TFE/HFP/VdF copolymer may further contain 0 to 20% by mol of another monomer. As the another monomer, preferable is at least one selected from the group consisting of ethylenically unsaturated monomers (excluding TFE, HFP and VdF) represented by the above formulas (5) and (6); more preferable is at least one selected from the group consisting of fluorine-containing monomers such as perfluoro(methyl vinyl ether), perfluoro(ethyl vinyl ether), perfluoro(propyl vinyl ether), chlorotrifluoroethylene, 2-chloropentafluoropropene and perfluorinated vinyl ethers (for example, perfluoroalkoxy vinyl ethers such as $CF_3OCF_2CF_2CF_2OCF=CF_2$), perfluoroalkyl vinyl ethers, perfluoro-1,3-butadiene, trifluoroethylene, hexafluoroisobutene, vinyl fluoride, ethylene, propylene, $CF_2=CHBr$, $CH_2=CH-CF_2CF_2Br$, $CF_2=CFBr$, $CH_2=CH-CF_2Br$ and alkyl vinyl ethers; and most preferable are perfluoro(methyl vinyl ether), perfluoro(ethyl vinyl ether) and perfluoro(propyl vinyl ether).

The tube in the present disclosure can be produced by molding the fluoropolymer into a tube shape. A method of molding the fluoropolymer into a tube shape is not limited, and the tube can be produced by melt extruding the fluoropolymer by using an extruding machine. Specifically, by using an extruding machine equipped with a cylinder, a screw, a die head and a die, the fluoropolymer is made into a melt state in the cylinder; the fluoropolymer in the melt state is extruded in a tube shape through the die by rotation of the screw; whereby the tube in the present disclosure is produced.

In the present disclosure, since as the fluoropolymer, one having a melt flow rate of 3 to 150 g/10 min is used, even in the case where the fluoropolymer is extruded into a tube shape by such melt extrusion, the generation of melt fracture on the outer surface and the inner surface of the tube can be suppressed; and there can thereby effectively be suppressed the increase in the visibility and the degradation of the low visibility due to the light refraction caused by irregularities by the generation of the melt fracture. The tube low in visibility can thereby be provided. In particular, according to the present disclosure, even in the case where molding into the tube by melt extrusion is carried out in a relatively high speed (for example, at a take-up speed of about 2 to 30 m/min), the generation of the melt fracture can effectively be suppressed and the tube low in visibility can be produced in high productivity.

The outer diameter of the tube in the present disclosure is not limited, and is preferably 0.5 to 5.0 mm and more preferably 1.0 to 3.0 mm. The thickness of the tube in the present disclosure is not limited, and is preferably 0.05 to 0.8 mm and more preferably 0.1 to 0.6 mm.

The surface roughness Ra of the inner surface of the tube in the present disclosure is, for example, 0.5 $\mu$m or smaller, preferably 0.2 $\mu$m or smaller and more preferably 0.16 $\mu$m or smaller, and may be 0.01 $\mu$m or larger. The surface roughness can be measured according to JIS B0601-1994.

The tube in the present disclosure is suitably used for transferring a liquid having a refractive index of 1.35 to 1.41, and is, since being low in visibility, suitably used for transferring a liquid fragrance. The tube in the present disclosure can further be suitably used as a tube for constituting a fragrance product equipped with a transparent container for accommodating a liquid having a refractive index of 1.35 to 1.41 and the tube for sucking up such a liquid; and in this case, the fragrance product can be made one in which the tube is substantially invisible, and can thereby be made the one excellent in the aesthetic property of its appearance. Further in the present disclosure, a container equipped with the tube in the present disclosure can also be provided, and such a container suitably includes containers for accommodating liquids having a refractive index of 1.35 to 1.41.

Hitherto, embodiments have been described, but it is to be understood that various changes and modifications in forms and details may be made without departing from the spirit and scope of the claims.

EXAMPLES

Then, the embodiments of the present disclosure will be described by way of Examples, but the present disclosure is not any more limited only to the Examples.

Respective numerical values in Examples were measured by the following methods.

<Melt flow rate (MFR)>

MFR of fluoropolymers used in Examples was determined by the following method. According to ASTM D1238 and by using a melt indexer (manufactured by Yasuda Seiki Seisakusho, Ltd.), there was determined the mass (g/10 min) of a copolymer flowing out per 10 min from its nozzle of 2.1 mm in inner diameter and 8 mm in length under a load of 5 kg. Here, the temperature in the measurement of the melt flow rate was determined by reference to the standard (ASTM D2116) for an individual fluoropolymer.

<Light transmittance at a wavelength of 300 nm>

The light transmittance at a wavelength of 300 nm of fluoropolymers used in Examples was determined by the following method. Pellets of a fluoropolymer were molded into a sheet shape of 0.1 mm in thickness, and the light transmittance at a wavelength of 300 nm of the obtained molded sheet was measured by using a spectrophotometer U-4000 (manufactured by Hitachi, Ltd.). Here, the fluoropolymer sheet was fabricated by the following method.

(Method of fabricating a fluoropolymer sheet)

Resin pellets were charged in a metal mold of 120 mm in diameter, set on a press machine heated at 300°C and melt pressed at a pressure of about 2.9 MPa to thereby obtain a fluoropolymer sheet of 0.1 mm in thickness.

<Refractive index>

The refractive index of fluoropolymers and a liquid fragrance used in Examples was measured at 25°C by using an Abbe's refractometer (manufactured by Atago Co., Ltd.) with the sodium D-line as its light source. The measurement of the refractive index of a fluoropolymer was carried out by using a fluoropolymer sheet fabricated by the above method.

<Haze value>

The haze value of fluoropolymers used in Examples was determined by the following method. Pellets of a fluoropolymer were molded into a sheet shape of 0.1 mm in thickness according to the method described above, and for the obtained molded sheet, the haze value was measured by using a haze meter (manufactured by Toyo Seiki Seisaku-sho Ltd., Haze-Gard II,) according to ASTM D1003.

<The number of fish eyes>

The number of fish eyes of fluoropolymers used in Examples was determined by the following method. By using pellets of a fluoropolymer, and by a single-layer film molding machine equipped with a T die, a film of the fluoropolymer was prepared under such molding conditions that the take-up speed was about 3 m/min and the width of the film became 70 mm and the thickness thereof became 0.05 to 0.06 mm (central part). Then, sampling of films for measurement was started from 30 min after the start of the molding, and films for measurement of 5 m in length were sampled.

Then, both edges of the obtained film for measurement were masked; and for a central part of 50 mm in width, detection of fish eyes was carried out by using a surface inspection apparatus (manufactured by Mitsubishi Rayon Co., Ltd., LSC-3100V); for detected fish eyes, the number of fish eyes having a size of 50 $\mu$m or larger in one side was measured and the number of the fish eyes per 1 $m^2$ was determined; and the number is taken as the number of fish eyes (unit: the number/$m^2$) of the fluoropolymer.

Here, the molding of the film for measurement and the measurement of fish eyes were carried out carefully so that there was no contamination of foreign matter such as dirt and dusts, and in a Class 1000 cleanroom (the number of particulates of 0.5 $\mu$m or larger in air of 1 $ft^3$ (cubic feet) is 1,000 or less).

<Yellow index>

The yellow index of fluoropolymers used in Example was determined by the following method. Pellets of a fluoropolymer were filled in a dedicated cell of a colorimeter (manufactured by Nippon Denshoku Industries Co., Ltd., ZE-6000), and measurement of yellow index was carried out by using the colorimeter according to JIS K7373, and the obtained value was taken as the yellow index.

<Crystallinity>

The crystallinity of fluoropolymers used in Examples was determined by the following method. On a fluoropolymer, wide-angle X-ray diffractometry was carried out at an output of 40 kV-40 mA in the range of scanning angles of 5 to 30° by using an X-ray diffractometer (manufactured by Rigaku Corp., SmartLab). Then, from the obtained measurement, by using analysis software (manufactured by Rigaku Corp., JADE6.0), a peak area derived from a crystal of the fluoropolymer and a whole peak area were calculated and the crystallinity of the fluoropolymer was determined by the following expression.

$$\text{Crystallinity of the fluoropolymer (\%)} = 100 \times \frac{\text{(the peak area derived from a crystal of the fluoropolymer)}}{\text{(the whole peak area)}}$$

<Tensile elastic modulus>

The tensile elastic modulus of fluoropolymers used in Examples was determined by the following method. Pellets of a fluoropolymer was set in a metal mold, held at 240 to 300°C for 15 to 30 min on a heat press machine to melt the polymer, and thereafter loaded with a load of 3 MPa for 1 min to be compression molded to thereby fabricate a sheet specimen of 2 mm in thickness. Then, the sheet specimen was punched out by using an ASTM D638 Type V dumbbell to thereby obtain a dumbbell specimen with a distance between marked lines of 3.18 mm. The tensile elastic modulus at 25°C of the obtained dumbbell specimen was measured by using an Autograph (manufactured by Shimadzu Corp., AGS-J 5kN) according to ASTM D638 under the condition of 50 mm/min.

<Content of each of Na, Cu, K, Ca, Fe and Zn>

The ashing analysis of fluoropolymers were carried out by an ashing method described in International Publication No. WO94/28394. That is, a sample was precisely weighed in the range of 2 to 6 mg from pellets of a fluoropolymer used in Examples, heated in a graphite cuvette at 1,100°C for 180 sec to be ashed, and analyzed by an atomic absorption spectrophotometer (a polarized Zeeman atomic absorption spectrophotometer (Z-8100, manufactured by Hitachi, Ltd.)).
In Examples, the above ashing method was used, but as required, an ashing analysis method different from this may be used. For example, the following method can be used. That is, 1 g of a sample was precisely weighed, put in a platinum crucible (platinum purity: 99.9%), and ashed by a gas burner or ashed at 500°C for 30 min in an electric oven; and thereafter, an ash content remaining in the platinum crucible was dissolved in a 35% hydrochloric acid to thereby obtain a solution. For the obtained solution, metal contents were measured by using an ICP atomic emission analyzer (SPS300, manufactured by Seiko Instruments Inc.) or a flameless atomic absorption spectrophotometer.
<Initial pyrolysis temperature>
The initial pyrolysis temperature of fluoropolymers used in Examples was measured. A fluoropolymer was heated in an air atmosphere and at a temperature-rise rate of 10°C/min by using a differential thermal·thermogravimetric analyzer, TG/DTA 6200 or TG/DTA 7200 (manufactured by Hitachi High-Tech Corp.); and a temperature when 1% of the mass of the fluoropolymer was lost was taken as the initial pyrolysis temperature.

<Surface roughness Ra>

Specimens were fabricated by cutting tubes obtained in Examples, and there was measured the surface roughness Ra of positions of each specimen corresponding to the inner surface and the outer surface of a tube. The measurement of five measurement points was repeated three times by using a surface roughness tester (manufactured by Mitsutoyo Corp., SURFTESTSV-600) according to JIS B0601-1994, and the average value of the obtained measurement values was taken as the surface roughness Ra.

Example 1

Pellets of a fluoropolymer were extruded at a take-up speed of 8 m/min by using an extruding machine (cylinder shaft diameter: 20 mm, L/D = 24) to thereby obtain a tube having an outer diameter of 2.0 mm, an inner diameter of 1.2 mm, a surface roughness Ra of the tube inner surface of 0.08 $\mu$m and a surface roughness Ra of the tube outer surface of 0.05 $\mu$m. The temperatures of a cylinder and a die of the extruding machine were set at 160 to 240°C.

Here, in Example 1, an ethylene [Et]/tetrafluoroethylene [TFE]/hexafluoropropylene [HFP]/2,3,3,4,4,5,5-heptafluoro-1-pentene ($CH_2=CFCF_2CF_2CF_2H$) [H2P] copolymer was used as the fluoropolymer. The composition of the Et/TFE/HFP/H2P copolymer used was 44.5% by mol of an Et unit, 40.5% by mol of a TFE unit, 14.5% by mol of an HFP unit and 0.5% by mol of an H2P unit, and had a melt flow rate (at 265°C, under a load of 5 kg) of 40 g/10 min, a light transmittance at a wavelength of 300 nm of 92%, a refractive index of 1.383, a haze value of 0.8%, the number of fish eyes of $323/m^2$, a yellow index of -6, a tensile elastic modulus of 950 MPa, a crystallinity of 22%, an initial pyrolysis temperature of 357°C, and a content of each of Na, Cu, K, Ca, Fe and Zn of lower than 0.5 $\mu$g/1 g as measured by the ashing method.

Then, the tube thus obtained was immersed in a liquid fragrance (refractive index: 1.38), Victoria's Secret, Bombshell Seduction, manufactured by Eau De Parfum Spray Co.; when the container was visually carefully observed from a distance of 50 cm from the front of the container with the background color of R: 247, G: 208 and B: 169 in RGB values, the presence of the tube was not confirmed, and by visually carefully observing the container from an approached distance of 20 cm, the presence of the tube was confirmed dimly. From this result, it can be said that the tube obtained in Example 1, when being immersed in liquids having a refractive index of 1.38, is low in visibility, and when being applied to liquid fragrance products and the like, is viewed as being tubeless at first sight, and can be made to be in the state of being invisible unless cautiously viewed, whereby the tube can be excellent in the aesthetic property of its appearance.

Example 2

There was obtained a tube having an outer diameter of 2.0 mm, an inner diameter of 1.2 mm, a surface roughness Ra of the tube inner surface of 0.16 $\mu$m and a surface roughness Ra of the tube outer surface of 0.14 $\mu$m, as in Example 1, except for using, as a fluoropolymer, a copolymer (a copolymer having the same composition in Example 1, and having a light transmittance at a wavelength of 300 nm of 91%, a refractive index of 1.383, a haze value of 1.2%, the number of fish eyes of $831/m^2$, a yellow index of -3, a tensile elastic modulus of 900 MPa, a crystallinity of 18%, an initial pyrolysis temperature of 375°C, and a content of each of Na, Cu, K, Ca, Fe and Zn of lower than 0.5 $\mu$g/1 g as measured by the ashing method) having a melt flow rate (at 265°C, under a load of 5 kg) of 5.5 g/10 min. Then, as in Example 1, the tube was immersed in the liquid fragrance in a container; when the container was visually carefully observed from a distance of 50 cm from the front of the container with the same background color as in Example 1, the presence of the tube was not confirmed, and by visually carefully observing the container from an approached distance of 20 cm, the presence of the tube was confirmed dimly. From this result, it can be said that the tube obtained in Example 2, when being immersed in liquids having a refractive index of 1.38, is low in visibility, and when being applied to liquid fragrance products and the like, is viewed as being tubeless at first sight, and can be made to be in the state of being invisible unless cautiously viewed, whereby the tube can be excellent in the aesthetic property of its appearance.

Example 3

Pellets of a fluoropolymer were extruded at a take-up speed of 8 m/min by using an extruding machine (cylinder shaft diameter: 20 mm, L/D = 24) to thereby obtain a tube having an outer diameter of 2.0 mm, an inner diameter of 1.2 mm, a surface roughness Ra of the tube inner surface of 0.08 $\mu$m and a surface roughness Ra of the tube outer surface of 0.06 $\mu$m. The temperatures of a cylinder and a die of the extruding machine were set at 240 to 290°C.

Here, in Example 3, a CTFE/TFE/PPVE copolymer was used as the fluoropolymer. The composition of the CTFE/TFE/PPVE copolymer used was 21.3% by mol of a CTFE unit, 76.3% by mol of a TFE unit and 2.4% by mol of a PPVE unit, and had a melt flow rate (at 297°C, under a load of 5 kg) of 30 g/10 min, a light transmittance at a wavelength of 300 nm of 95%, a refractive index of 1.373, a haze value of 1.5%, the number of fish eyes of $463/m^2$, a yellow index of -20, a tensile elastic modulus of 580 MPa, a crystallinity of 32%, an initial pyrolysis temperature of 415°C, and a content of each of Na, Cu, K, Ca, Fe and Zn of lower than 0.5 $\mu$g/1 g as measured by the ashing method.

Then, the tube thus obtained was immersed in a liquid fragrance (refractive index: 1.38), Victoria's Secret, Bombshell Seduction, manufactured by Eau De Parfum Spray Co.; when the container was visually carefully observed from a distance of 100 cm from the front of the container with the background color of R: 247, G: 208 and B: 169 in RGB values, the presence of the tube was not confirmed, and by visually carefully observing the container from an approached distance of 50 cm, the presence of the tube was confirmed dimly. From this result, it can be said that the tube obtained in Example 3, when being immersed in liquids having a refractive index of 1.38, is low in visibility, and when being applied to liquid fragrance products and the like, is viewed as being tubeless at first sight, and can be made to be in the state of being invisible unless cautiously viewed, whereby the tube can be excellent in the aesthetic property of its appearance.

Example 4

Pellets of a fluoropolymer were extruded at a take-up speed of 3 m/min by using an extruding machine (cylinder shaft diameter: 20 mm, L/D = 24) to thereby obtain a tube having an outer diameter of 2.0 mm, an inner diameter of 1.2 mm, a surface roughness Ra of the tube inner surface of 0.14 $\mu$m and a surface roughness Ra of the tube outer surface of 0.10 $\mu$m. The temperatures of a cylinder and a die of the extruding machine were set at 280 to 340°C.

Here, in Example 4, a TFE/HFP/VdF copolymer was used as the fluoropolymer. The composition of the TFE/HFP/VdF copolymer used was 84.5% by mol of a TFE unit, 7.0% by mol of an HFP unit, 8.0% by mol of a VdF unit and 0.5% by mol of a PPVE unit, and had a melt flow rate (at 265°C, under a load of 5 kg) of 3 g/10 min, a light transmittance at a wavelength of 300 nm of 87%, a refractive index of 1.37, a haze value of 3.8%, the number of fish eyes of 4,185/m$^2$, a yellow index of 5, a tensile elastic modulus of 450 MPa, a crystallinity of 39%, an initial pyrolysis temperature of 400°C, and a content of each of Na, Cu, K, Ca, Fe and Zn of lower than 0.5 $\mu$g/1 g as measured by the ashing method.

Then, the tube thus obtained was immersed in the liquid fragrance (refractive index: 1.38), Victoria's Secret, Bombshell Seduction, manufactured by Eau De Parfum Spray Co.; when the container was visually carefully observed from a distance of 150 cm from the front of the container with the background color of R: 247, G: 208 and B: 169 in RGB values, the presence of the tube was not confirmed, and by visually carefully observing the container from an approached distance of 100 cm, the presence of the tube was confirmed dimly. From this result, it can be said that the tube obtained in Example 4, when being immersed in liquids having a refractive index of 1.38, is low in visibility, and when being applied to liquid fragrance products and the like, is viewed as being tubeless at first sight, and can be made to be in the state of being invisible unless cautiously viewed, whereby the tube can be excellent in the aesthetic property of its appearance.

Example 5

Pellets of a fluoropolymer were extruded at a take-up speed of 5 m/min by using an extruding machine (cylinder shaft diameter: 20 mm, L/D = 24) to thereby obtain a tube having an outer diameter of 2.0 mm, an inner diameter of 1.2 mm, a surface roughness Ra of the tube inner surface of 0.07 $\mu$m and a surface roughness Ra of the tube outer surface of 0.05 $\mu$m. The temperatures of a cylinder and a die of the extruding machine were set at 120 to 230°C.

Here, in Example 5, a TFE/HFP/VdF copolymer was used as the fluoropolymer. The composition of the TFE/HFP/VdF copolymer used was 34.5% by mol of a TFE unit, 5.0% by mol of an HFP unit and 60.5% by mol of a VdF unit, and had a melt flow rate (at 230°C, under a load of 2.16 kg) of 20 g/10 min, a light transmittance at a wavelength of 300 nm of 85%, a refractive index of 1.375, a haze value of 3.0%, the number of fish eyes of 1,358/m$^2$, a yellow index of -3, a tensile elastic modulus of 150 MPa, a crystallinity of 15%, an initial pyrolysis temperature of 374°C, and a content of each of Na, Cu, K, Ca, Fe and Zn of lower than 0.5 $\mu$g/1 g as measured by the ashing method.

Then, the tube thus obtained was, as in Example 1, immersed in the liquid fragrance in a container; when the container was visually carefully observed from a distance of 80 cm from the front of the container with the same background color as in Example 1, the presence of the tube was not confirmed, and by visually carefully observing the container from an approached distance of 50 cm, the presence of the tube was confirmed dimly. From this result, it can be said that the tube obtained in Example 5, when being immersed in liquids having a refractive index of 1.38, is low in visibility, and when being applied to liquid fragrance products and the like, is viewed as being tubeless at first sight, and can be made to be in the state of being invisible unless cautiously viewed, whereby the tube can be excellent in the aesthetic property of its appearance.

**Claims**

1.   A tube for transferring a liquid having a refractive index of 1.35 to 1.41, the tube comprising a fluoropolymer wherein the fluoropolymer has a melt flow rate of 3 to 150 g/10 min and a light transmittance at a wavelength of 300 nm of 85% or higher.

2.   The tube according to claim 1, wherein the fluoropolymer has a refractive index of 1.37 to 1.39.

3.   The tube according to claim 1 or 2, wherein the fluoropolymer has a haze value of 0.01 to 5.0%.

4.   The tube according to any one of claims 1 to 3, wherein the fluoropolymer has a number of fish eyes of 5,000/m$^2$ or less.

5.   The tube according to any one of claims 1 to 4, wherein the fluoropolymer has a yellow index value of 5 or lower.

6.   The tube according to any one of claims 1 to 5, wherein the fluoropolymer has a tensile elastic modulus of 400 MPa or higher.

7. The tube according to any one of claims 1 to 6, wherein the tube has an outer diameter of 0.5 to 5.0 mm.

8. The tube according to any one of claims 1 to 7, wherein the fluoropolymer is at least one selected from the group consisting of an ethylene/tetrafluoroethylene/hexafluoropropylene copolymer, a polychlorotrifluoroethylene, a chlorotrifluoroethylene-based copolymer, a vinylidene fluoride/tetrafluoroethylene copolymer, a tetrafluoroethylene/hexafluoropropylene copolymer, and a tetrafluoroethylene/hexafluoropropylene/vinylidene fluoride copolymer.

9. The tube according to any one of claims 1 to 8, wherein the fluoropolymer is an ethylene/tetrafluoroethylene/hexafluoropropylene copolymer.

10. The tube according to any one of claims 1 to 9, wherein the fluoropolymer has a content of each of Na, Cu, K, Ca, Fe and Zn of 1.0 $\mu$g/1 g or lower as measured by an ashing method.

11. The tube according to any one of claims 1 to 10, wherein the liquid is a liquid fragrance.

12. A container, comprising the tube according to any one of claims 1 to 11.

13. A fragrance product, comprising a transparent container accommodating the liquid having a refractive index of 1.35 to 1.41, and the tube according to any one of claims 1 to 12 for sucking up the liquid.

## INTERNATIONAL SEARCH REPORT

| | International application No. |
|---|---|
| | PCT/JP2020/035090 |

### A. CLASSIFICATION OF SUBJECT MATTER
A45D 34/02(2006.01)i; F16L 11/06(2006.01)i
FI: F16L11/06; A45D34/02 510Z
According to International Patent Classification (IPC) or to both national classification and IPC

### B. FIELDS SEARCHED
Minimum documentation searched (classification system followed by classification symbols)
A45D34/02; F16L11/06; B65D83/00; B05B15/30

| Documentation searched other than minimum documentation to the extent that such documents are included in the fields searched | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2014-12185 A (MEADWESTVACO CORPORATION) 23 January 2014 (2014-01-23) paragraphs [0012]-[0015], [0018], [0025], fig. 5 | 1-13 |
| Y | JP 2010-121126 A (JAPAN POLYPROPYLENE CORPORATION) 03 June 2010 (2010-06-03) paragraph [0030] | 1-13 |
| Y | JP 2011-33643 A (TOPPAN PRINTING CO., LTD.) 17 February 2011 (2011-02-17) paragraph [0060] | 1-13 |
| A | JP 2009-35272 A (YOSHINO KOGYOSHO CO., LTD.) 19 February 2009 (2009-02-19) paragraphs [0023]-[0026], fig. 1 | 1-13 |
| A | US 8646245 B2 (APTAR FRANCE SAS) abstract | 1-13 |

☐ Further documents are listed in the continuation of Box C.  ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 10 November 2020 (10.11.2020) | 24 November 2020 (24.11.2020) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**
Information on patent family members

International application No.

PCT/JP2020/035090

| Patent Documents referred in the Report | Publication Date | Patent Family | Publication Date |
|---|---|---|---|
| JP 2014-12185 A | 23 Jan. 2014 | WO 2007/047168 A2 paragraphs [0019]-[0024], [0028], [0035], fig. 5 KR 10-2008-0073705 A CN 101321590 A | |
| JP 2010-121126 A | 03 Jun. 2010 | (Family: none) | |
| JP 2011-33643 A | 17 Feb. 2011 | (Family: none) | |
| JP 2009-35272 A | 19 Feb. 2009 | (Family: none) | |
| US 8646245 B2 | | WO 2008/155494 A1 FR 2917650 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014012185 A

- WO 9428394 A